# EUROPEAN PATENT SPECIFICATION

(11) **EP 2 890 264 B1**
(45) Date of publication and mention of the grant of the patent: **21.11.2018**
(21) Application number: 13847477.0
(22) Date of filing: 11.10.2013
(51) Int. Cl.: A43D 1/08

(54) **SYSTEM INCLUDING FOOTWEAR AND SOCK HAVING ALIGNING INDICIA**
SYSTEM MIT SCHUHWERK UND SOCKE MIT AUSRICHTUNGSMARKIERUNGEN
SYSTÈME COMPRENANT UNE CHAUSSURE ET UNE CHAUSSETTE PRÉSENTANT DES REPÈRES ALIGNÉS

(30) Priority: 15.10.2012 US 201213651990
(43) Date of publication of application: 08.07.2015
(73) Proprietor: NIKE Innovate C.V., Beaverton, OR 97005-6453 (US)
(72) Inventor: SPANKS, Jeffrey C., Beaverton, OR 97005-6453 (US); PANIAN, Nadia M., Beaverton, OR 97005-6453 (US); AVAR, Eric P., Beaverton, OR 97005-6453 (US)
(74) Representative: Müller-Boré & Partner Patentanwälte PartG mbB
(86) International application number: PCT/US2013/064489
(87) International publication number: WO 2014/062492

(56) References cited:
- EP-A1- 2 052 635
- US-A- 5 393 372
- US-A1- 2002 020 082
- US-A1- 2009 019 731
- Anonymous: "Back to Basics: Which socks do I wear with these?", effortlessgent , 5 January 2012 (2012-01-05), XP002732305, Retrieved from the Internet: URL:http://effortlessgent.com/back-to-basi cs-which-socks-do-i-wear-with-these/ [retrieved on 2014-11-10]
- Joshua Davis: "Do I match my dress socks to my shoes or my pants?", , 22 December 2013 (2013-12-22), XP002732306, Retrieved from the Internet: URL:with-josh.com/blog/2013/7/21/matching- your-socks [retrieved on 2014-11-10]

## Description

### FIELD OF THE INVENTION

The present invention relates generally to a footwear and sock system.

### BACKGROUND

It is advantageous to include markings on footwear to facilitate observation, for example, by a trainer or by still photography or video motion capture for analysis of athletic performance. For example, systems have been developed that include markings on various external surfaces of footwear to provide references for the positioning of the foot of the wearer during athletic movements. Markings are also used for aesthetic purposes to provide footwear with a desired appearance.

US 2009/019731 A1 discloses a method for displaying content in a shoe system. The shoe has different, customizable appearances depending upon the content.

### SUMMARY

The present invention is directed to a footwear system according to appended independent claim 1. Preferred embodiments are defined in the dependent claims.

### BRIEF DESCRIPTION OF THE DRAWINGS

The invention can be better understood with reference to the following drawings and description. The components in the figures are not necessarily to scale, emphasis instead being placed upon illustrating the principles of the invention. Moreover, in the figures, like reference numerals designate corresponding parts throughout the different views.
FIG. 1 shows an assembled view of an exemplary footwear system;
FIG. 2 shows an exploded view of the footwear system shown in FIG. 1;
FIG. 3 shows another assembled view of the footwear system shown in FIG. 1;
FIG. 4 shows another assembled view of the footwear system shown in FIG. 1;
FIG. 5 shows an assembled view of an exemplary footwear system;
FIG. 6 shows an assembled view of an exemplary footwear system;
FIG. 7 shows an assembled view of an exemplary footwear system;
FIG. 8 shows the sock from the system shown in FIG. 7 with a strap portion in a fastened condition;
FIG. 9 shows the sock from the system shown in FIG. 7 with a strap portion in an unfastened condition;
FIG. 10 is a front, assembled view of a footwear system;
FIG. 11 is a rear view of an assembled footwear system featuring both left and right socks and footwear;
FIG. 12 is an exploded view of a footwear system having interchangeable socks;
FIGS. 13-15 are assembled views of the system shown in FIG. 12;
FIG. 16 shows a footwear system having interchangeable footwear; and
FIG. 17 shows another footwear system having interchangeable footwear.

### DETAILED DESCRIPTION

The following discussion and accompanying figures disclose a sole structure for an article of footwear. Concepts associated with the footwear disclosed herein may be applied to a variety of athletic footwear types, including soccer shoes, baseball shoes, football shoes, golf shoes, and hiking shoes and boots, for example. Accordingly, the concepts disclosed herein apply to a wide variety of footwear types.

For consistency and convenience, directional adjectives are employed throughout this detailed description corresponding to the illustrated embodiments. The term "longitudinal," as used throughout this detailed description and in the claims, refers to a direction extending a length of a sole structure, i.e., extending from a forefoot portion to a heel portion of the sole. The term "forward" is used to refer to the general direction in which the toes of a foot point, and the term "rearward" is used to refer to the opposite direction, i.e., the direction in which the heel of the foot is facing.

The term "lateral direction," as used throughout this detailed description and in the claims, refers to a side-to-side direction extending a width of a sole. In other words, the lateral direction may extend between a medial side and a lateral side of an article of footwear, with the lateral side of the article of footwear being the surface that faces away from the other foot, and the medial side being the surface that faces toward the other foot.

The term "lateral axis," as used throughout this detailed description and in the claims, refers to an axis oriented in a lateral direction.

The term "horizontal," as used throughout this detailed description and in the claims, refers to any direction substantially parallel with the ground, including the longitudinal direction, the lateral direction, and all directions in between. Similarly, the term "side," as used in this specification and in the claims, refers to any portion of a component facing generally in a lateral, medial, forward, and/or rearward direction, as opposed to an upward or downward direction.

The term "vertical," as used throughout this detailed description and in the claims, refers to a direction generally perpendicular to both the lateral and longitudinal directions. For example, in cases where a sole is planted flat on a ground surface, the vertical direction may extend from the ground surface upward. It will be understood that each of these directional adjectives may be applied to individual components of a sole. The term "upward" refers to the vertical direction heading away from a ground surface, while the term "downward" refers to the vertical direction heading towards the ground surface. Similarly, the terms "top," "upper," and other similar terms refer to the portion of an object substantially furthest from the ground in a vertical direction, and the terms "bottom," "lower," and other similar terms refer to the portion of an object substantially closest to the ground in a vertical direction.

For purposes of this disclosure, the foregoing directional terms, when used in reference to an article of footwear, shall refer to the article of footwear when sitting in an upright position, with the sole facing groundward, that is, as it would be positioned when worn by a wearer standing on a substantially level surface.

In addition, for purposes of this disclosure, the term "fixedly attached" shall refer to two components joined in a manner such that the components may not be readily separated (for example, without destroying one or both of the components). Exemplary modalities of fixed attachment may include joining with permanent adhesive, rivets, stitches, nails, staples, welding or other thermal bonding, and/or other joining techniques. In addition, two components may be "fixedly attached" by virtue of being integrally formed, for example, in a molding process.

The term "indicia," as used throughout this detailed description and in the claims, can refer to both singular and a plurality of markings. The markings forming indicia in the disclosed embodiments may have any of a variety of forms including, for example, shapes, logos, alpha-numeric characters, and/or other types of markings.

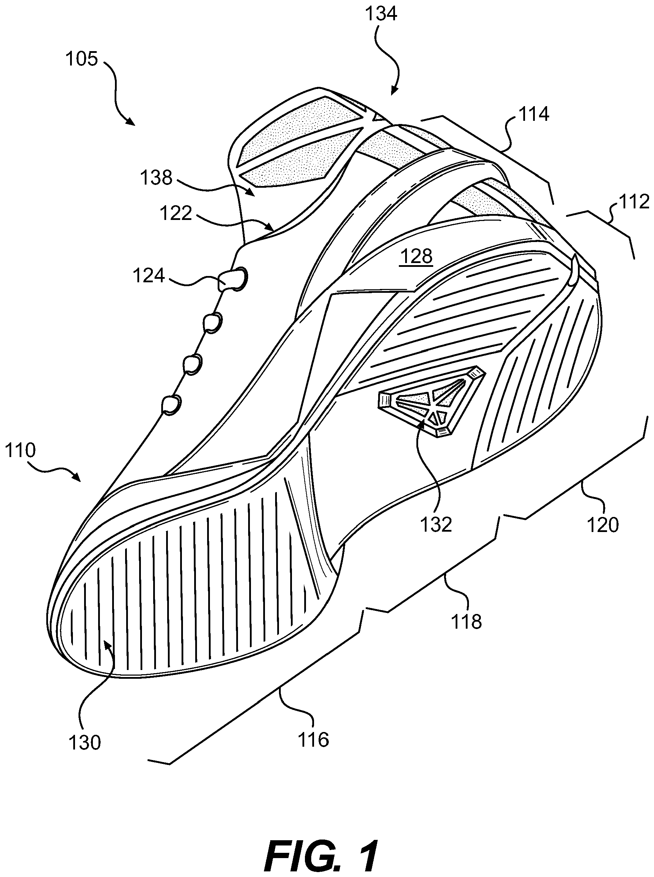

FIG. 1 depicts an embodiment of a footwear system 105. System 105 may include an article of footwear 110, which may include a sole structure 112 and an upper 114 secured to sole structure 112 and configured to receive a foot. System 105 may also include a sock 138. As discussed in greater detail below, footwear 110 and sock 138 may each have portions of indicia that, when sock 138 is worn by a wearer on a foot received within footwear 110, the indicia portions may align with one another to form the indicia.

For reference purposes, system 105 may be divided into three general regions: a forefoot region 116, a midfoot region 118, and a heel region 120. Forefoot region 116 generally includes portions of footwear 110 corresponding with the toes and the joints connecting the metatarsals with the phalanges. Midfoot region 118 generally includes portions of footwear 110 corresponding with an arch area of the foot. Heel region 120 generally corresponds with rear portions of the foot, including the calcaneus bone. Forefoot region 116, midfoot region 118, and heel region 120 are not intended to demarcate precise areas of footwear 10. Rather, forefoot region 116, midfoot region 118, and heel region 120 are intended to represent general relative areas of system 105.

Since various features of sock 138 and footwear 110 extend substantially the entire length of system 105, the terms forefoot region 116, midfoot region 118, and heel region 120 apply not only to footwear 110 and sock 138 in general, but also to the various features of footwear 110 and sock 138.

As shown in FIG. 1, upper 114 may include one or more material elements, which may be stitched, adhesively bonded, molded, or otherwise formed to define an interior void configured to receive a foot. The material elements may be selected and arranged to selectively impart properties such as durability, air-permeability, wear-resistance, flexibility, and comfort. Upper 114 may define an opening 122 configured to receive a foot of a wearer. In addition, upper 114 may include a lace 124, which may be utilized to modify the dimensions of the interior void, thereby securing the foot within the interior void and facilitating entry and removal of the foot from the interior void. Lace 124 may extend through apertures (eyelets) in upper 120. Upper 114 may alternatively implement any of a variety of other configurations, materials, and/or closure mechanisms. For example, alternative closure mechanisms, such as hook and loop fasteners (for example, straps), buckles, clasps, cinches, or any other arrangement for securing a foot within the void defined by upper 114.

Sole structure 112 may be fixedly attached to upper 114 (for example, with adhesive, stitching, welding, and/or other suitable techniques) and may have a configuration that extends between upper 114 and the ground. Sole structure 112 may include provisions for attenuating ground reaction forces (that is, cushioning and stabilizing the foot during vertical and horizontal loading). In addition, sole structure 112 may be configured to provide traction, impart stability, and/or limit various foot motions, such as pronation, supination, and/or other motions.

The configuration of sole structure 112 may vary significantly according to one or more types of ground surfaces on which sole structure 112 may be used. For example, the disclosed concepts may be applicable to footwear configured for use on indoor surfaces and/or outdoor surfaces. The configuration of sole structure 112 may vary based on the properties and conditions of the surfaces on which footwear 110 is anticipated to be used. For example, sole structure 112 may vary depending on whether the surface is harder or softer. In addition, sole structure 112 may be tailored for use in wet or dry conditions.

In some embodiments, sole structure 112 may be configured for a particularly specialized surface and/or condition. For example, in some embodiments, footwear 110 is illustrated in the accompanying figures as a basketball or court type shoe and, accordingly, the illustrated sole structure 112 is configured for providing cushioning, stability, and traction on hard, smooth surfaces, such as hardwood floors. The proposed sock/footwear systems may be applicable to any kind of footwear, however. For example, in some configurations, sole structure 112 may include a sole for a soccer shoe configured to provide traction and stability on soft, natural turf surfaces and/or on hard, artificial turf surfaces.

In some embodiments, sole structure 112 may include multiple components, which may individually and/or collectively provide footwear 110 with a number of attributes, such as support, rigidity, flexibility, stability, cushioning, comfort, reduced weight, and/or other attributes. In some embodiments, sole structure 112 may include an insole/sockliner (not shown), a midsole 128, and a ground engaging sole component 130, as shown in FIG. 1. In some cases, however, one or more of these components may be omitted.

The insole may be disposed in the void defined by upper 114. The insole may extend through each of forefoot region 116, midfoot region 118, and heel region 120, and between the lateral and medial sides of footwear 10. Insole 127 may be formed of a deformable (for example, compressible) material, such as polyurethane foams, or other polymer foam materials. Accordingly, the insole may, by virtue of its compressibility, provide cushioning, and may also conform to the foot in order to provide comfort, support, and stability.

Midsole 128 may be fixedly attached to a lower area of upper 114 (for example, through stitching, adhesive bonding, thermal bonding (for example, welding), and/or other techniques), or may be integral with upper 114. Midsole 128 may extend through each of forefoot region 116, midfoot region 118, and heel region 120, and between the lateral and medial sides of footwear 110. In some embodiments, portions of midsole 128 may be exposed around the periphery of footwear 110, as shown in FIG. 1. In other embodiments, midsole 128 may be completely covered by other elements, such as material layers from upper 114.

Upper 114 may be formed from any suitable material. For example, upper 114 may be formed from textiles, foam, leather, synthetic leather, and other suitable materials. Midsole 128 may be formed from any suitable material having the properties described above, according to the activity for which footwear 110 is intended. In some embodiments, midsole 128 may include a foamed polymer material, such as polyurethane (PU), ethyl vinyl acetate (EVA), or any other suitable material that operates to attenuate ground reaction forces as sole structure 112 contacts the ground during walking, running, or other ambulatory activities. Sole component 130 of footwear 110 may be formed using any of the materials described in U.S. Patent No. 5,709,954 to Lyden et al., entitled "Chemical Bonding of Rubber to Plastic in Articles of Footwear".

Sock 138 may have any suitable configuration. For example, sock 138 may be a low-cut, mid-cut, or high-cut length. For instance, basketball socks may be relatively low-cut (for example, extending one or two inches above the ankle) or mid-cut (extending approximately mid-way up the lower leg) or high-cut (extending up to the knee). Soccer socks are typically high-cut as well. Further, sock 138 may be formed of any suitable materials, such as cottons, polyesters, nylons, and other such materials, and may have any suitable construction (for example woven/knit).

A sock may have indicia located at one or more locations. In addition to imparting a unique aesthetic to system 105 and enhancing enjoyment of the wearer, indicia may provide other advantages. For example, the indicia of system 105 may increase the visibility of the wearer in various lighting and environmental conditions. The colors and/or reflectivity of the indicia may be selected to provide desired visual effects. In addition, indicia may also be utilized on system 105 during product testing to enhance the visibility of areas of the sock, footwear, and/or the leg and foot of the wearer that are subjected to tensile, compression, bending, or twisting forces. For example, the indicia may improve the degree to which areas of these components may be captured with still image photography or video, such as high-speed film or other mediums that visually-capture performance data during biomechanical or other forms of testing.

In some embodiments, footwear 110 may have indicia, such as a logo, symbol, design, or other type of marking, included on various portions of the footwear. For example, a first indicia 132 may be located on a bottom of sole component 130. In some cases, the indicia may be reflective and/or otherwise facilitate image/video capture or general observation of athletic movement. In some embodiments, the same logo of sole indicia 132 may be provided on other portions of footwear 110, such as on the medial or lateral side, heel region, or in a toe region of footwear 110. In some embodiments, portions of an indicia may be provided adjacent the opening to the upper and on the sock proximate the ankle, such that the indicia portion on the sock aligns with the indicia portion on the footwear when the sock and footwear are worn by a wearer. For example, this sock/footwear combination may form a second indicia 134 In some cases, the second indicia 134 formed by the sock/footwear combination may be the same logo shown elsewhere on the footwear, such as first indicia 132, as shown in FIG. 1.

Embodiments where the indicia portion on the sock aligns with the indicia portion on the footwear to form a sock/footwear combination indicia may enable image/video capture as well as general observation of an enlarged portion of the lower extremity. For example, portions of the ankle and/or Achilles tendon may coincide with the sock indicia. This may also enable the presentation of significantly larger indicia than could be included on the sock or footwear alone. Further, a sock/footwear combination indicia may be configured to have a desired aesthetic appearance.

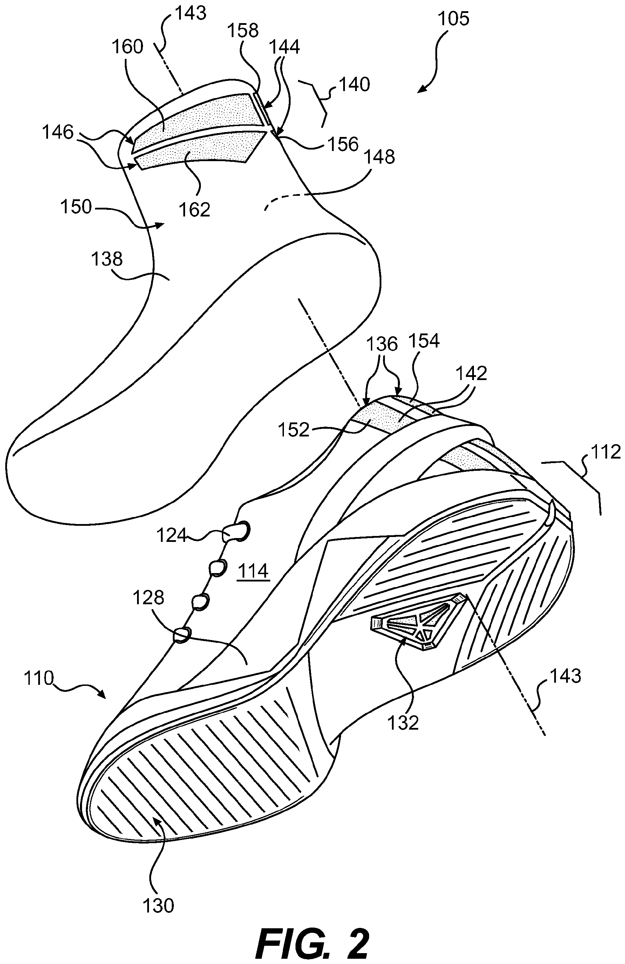

FIG. 2 shows an exploded view of system 105 from FIG. 1. System 105 may include a first indicia portion 136 disposed on upper 114 adjacent to opening 122. In addition, sock 138 may include a second indicia portion 140, which may be configured such that when sock 138 is worn by the wearer on a foot received within upper 114, one or more features of second indicia portion 140 may be substantially aligned with one or more features of first indicia portion 136 on upper 114 to form second indicia 134 (see Fig. 1).

The indicia may have any suitable configuration. For example, in some embodiments, the indicia may include three pair of elongate markings, wherein each pair of elongate markings includes a first marking and a second marking oriented substantially parallel to the first marking, each pair of elongate markings extending substantially radially from a central region. As shown in FIG. 2, first indicia portion 136 may include a first pair of elongate markings 142, including a first marking 152 and a second marking 154. In some embodiments, first marking 152 may be substantially parallel to second marking 154. As shown in FIG. 2, in some embodiments, first pair of markings 142 may be disposed on the rear portion of the heel region of upper 114 and may extend in a substantially vertical direction. For example, when viewed from the rear, first pair of elongate markings 142 may be in substantial alignment with a vertical axis 143. This arrangement may facilitate visualization and observation of portions of the footwear system, the leg, and or foot. For example, the radially oriented marking pairs may provide a cross-hairs effect, or a triangulation effect that may be more readily visible to the eye and/or in still photography or video capture equipment.

As also shown in FIG. 2, second indicia portion 140 disposed on sock 138 may include a second pair of elongate markings 144, which may include a third marking 156 and a fourth marking 158. Further, second indicia portion may also include a third pair of elongate markings 146, which may include a fifth marking 160 and a sixth marking 162.

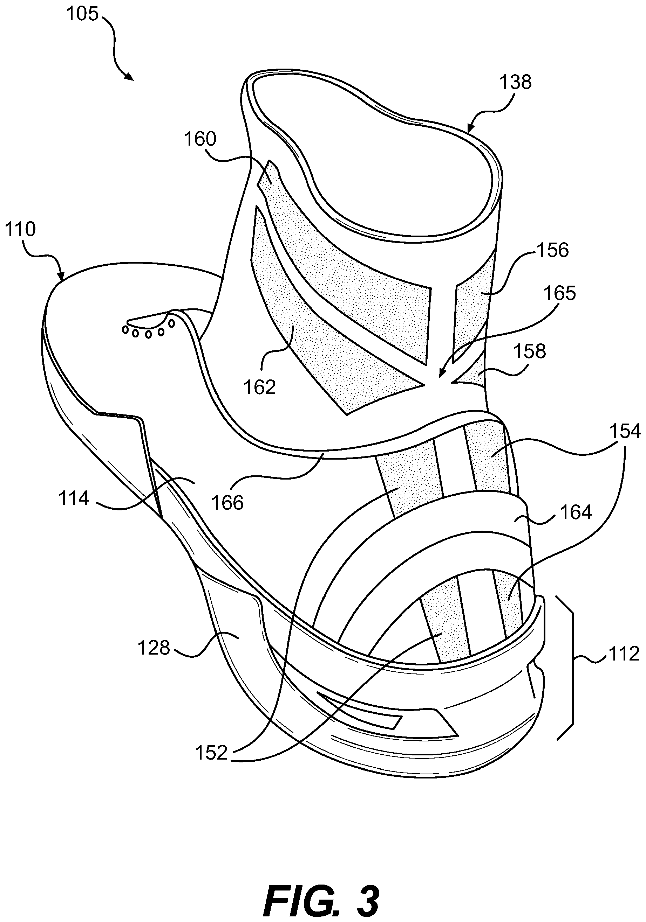

As shown in FIGS. 2-4, third marking 156 and fourth marking 158 may be substantially parallel to one another and may extend upward and toward a medial side 148 of sock 138. Further, fifth marking 160 and a sixth marking 162 may extend upward and toward a lateral side 150 of sock 138. As shown in FIG. 3, first marking 152, second marking 154, third marking 156, fourth marking 158, fifth marking 160, and sixth marking 162 may extend substantially radially from a central region 165.

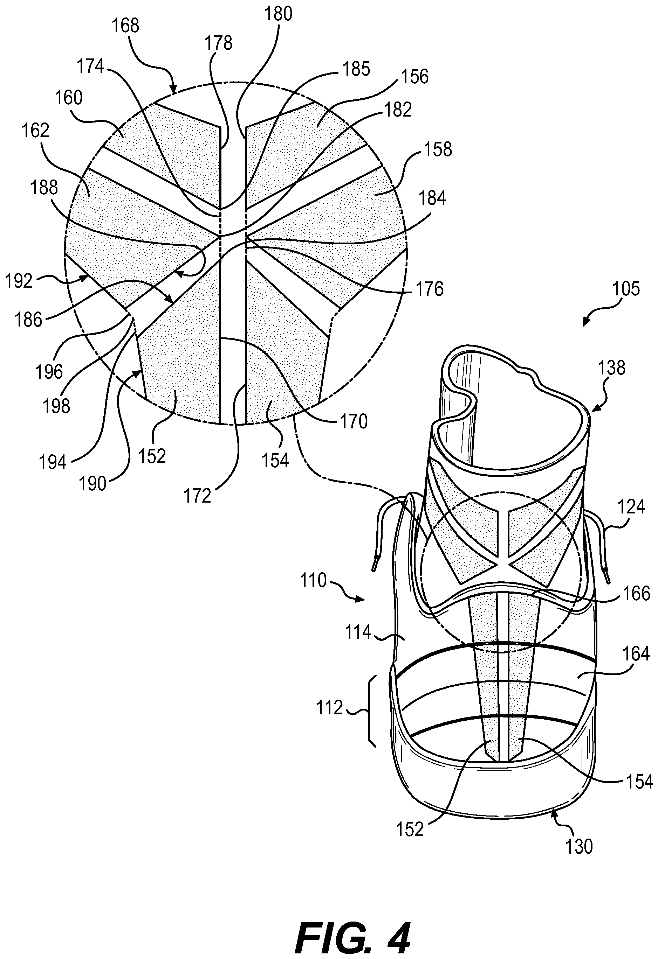

As shown in FIG. 3, footwear 110 may include additional features, such as a liner 166 disposed internally to upper 114. In some embodiments, a portion of liner 166 may be visible at the opening of footwear 10, as shown in FIG. 3. As also shown in FIG. 3, footwear 110 may also have additional structural components externally, such as a support piece 164 included on the heel portion of footwear 110. In some embodiments, indicia may be interrupted by such additional structural features. For example, as shown in FIG. 3, first marking 152 and second marking 154 may be interrupted by support piece 164. In other embodiments, the indicia may extend onto the additional structural features. For example, as shown in FIG. 4, in some embodiments, first marking 152 and second marking 154 may extend across support piece 164.

An enlarged view 168 in FIG. 4 illustrates a central portion of the sock/footwear combination indicia. Enlarged view 168 illustrates the portions of the indicia arranged without footwear components for purposes of showing the relative positioning and alignment of the indicia markings. For example, enlarged view 168 illustrates the substantial alignment of some indicia features and substantial non-alignment of other indicia features, when sock 138 is worn by a wearer on a foot received within footwear 110.

In some embodiments, when sock 138 is worn by a wearer on a foot received within footwear 110, one or more features of first marking 152 may be substantially aligned with one or more features of third marking 156 and/or fourth marking 158. For example, first marking 152 may include a first inner edge 170. Similarly, second marking 154 may include a second inner edge 172. As discussed above, first marking 152 and second marking 154 may be arranged substantially parallel to one another. Further, in some embodiments, first inner edge 170 may be substantially parallel to second inner edge 172.

In addition, when sock 138 is worn by a wearer on a foot received within footwear 110, one or more features of first marking 152 may be substantially aligned with one or more features of fifth marking 160 and/or sixth marking 162. For example, first inner surface 170 of first marking 152 may be in substantial alignment with a third surface 178 of fifth marking 160. This alignment is illustrated by a first axis 174 shown in FIG. 4 extending from a first vertex 184 of first marking 152, a second vertex of sixth marking 162, and a third vertex 182 of fifth marking 160, all of which may lie on axis 174. Similarly, second inner edge 172 of second marking 154 may be in substantial alignment with fourth edge 180, for example, along a second axis 176, as shown in FIG. 4.

In another manner of alignment, first marking 152 may have a fifth edge 186 and sixth marking 162 may have a sixth edge 188. When sock 138 is worn by a wearer on a foot received within footwear 110, fifth edge 186 and sixth edge 188 may be substantially aligned with one another. For example, as shown in FIG. 4, fifth edge 186 and sixth edge 188 may be substantially parallel to one another. Further, in some embodiments, fifth edge 186 and sixth edge 188 may face each other.

In some embodiments, features of indicia portions may be substantially non-aligned with features of other indicia portions. For example, first marking 152 may have a seventh edge 190 and sixth marking 162 may have an eighth edge 192. In some embodiments, when sock 138 is worn by a wearer on a foot received within footwear 110, seventh edge 190 and eighth edge 192 may be in substantial non-alignment. For example, as shown in FIG. 4, seventh edge 190 may be oriented along a third axis 194, and eighth edge 192 may be oriented along a fourth axis 196. As shown in FIG. 4, third axis 194 and fourth axis 196 may be substantially non-aligned and substantially non-parallel to each other. Accordingly, third axis 194 and fourth axis 196 may intersect at an intersection 198. In some embodiments, intersection 198 may be substantially equidistant from first marking 152 and sixth marking 162, as shown in FIG. 4.

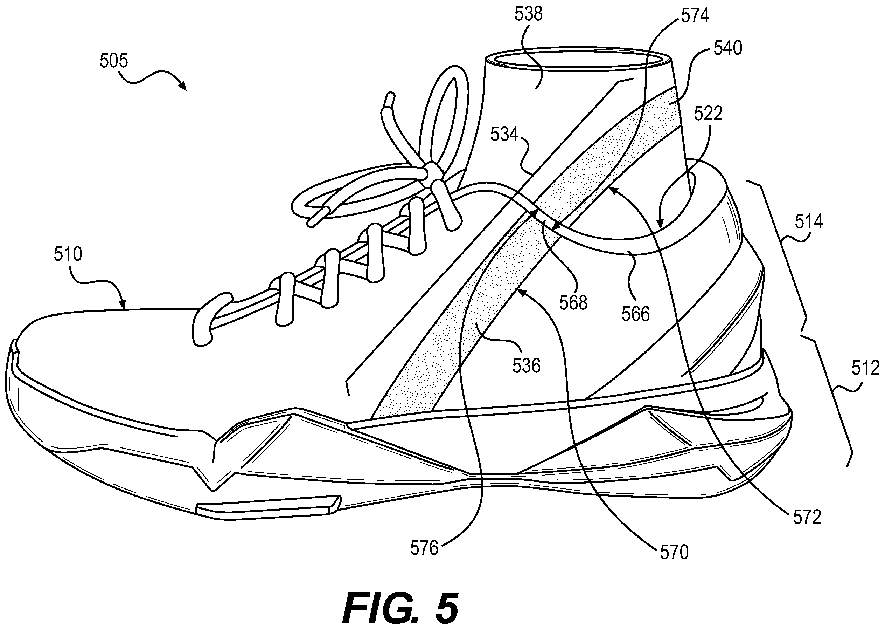

FIG. 5 shows a system 505 including an article of footwear 510 having a sole structure 512 secured to an upper 514. Upper 514 may define an opening 522 configured to receive the foot of a wearer. System 505 may also include a sock 538. When combined, markings on footwear 510 and sock 538 may form an indicia 534 formed by indicia portions on both footwear 510 and sock 538, as shown in FIG. 5. For example, upper 514 may include a first indicia portion 536, at least a portion of which may be disposed adjacent opening 522. Sock 538 may include a second indicia portion 540. When sock 538 is disposed on the foot of a wearer received within footwear 510, first indicia portion 536 may substantially align with second indicia 540 to form indicia 534.

As shown in FIG. 5, footwear 510 may include a liner 566, at portion of which may be visible in the area of opening 522. In some embodiments, liner 566 may have a color that differs from indicia 534, such that a small portion 568 of liner 566 interrupts indicia 534. In some embodiments, liner 522 may not be visible or may include coloration in portion 568 to provide continuity between first indicia portion 536 and second indicia portion 540. Further, in some embodiments, the different coloration and consequent discontinuity may form a portion of indicia 534.

Indicia 534 may have any suitable configuration. For example, indicia 534 may be disposed on a medial or lateral portion of system 505, as shown in FIG. 5. In other embodiments, indicia 534 may be located on other portions of system 505. Further, indicia 534 may have any suitable form. For example, in some embodiments, indicia 534 may be configured as a stripe, as also shown in FIG. 5.

First indicia portion 536 may include a first edge 570 and second indicia portion 540 may include a second edge 572. When sock 538 is worn by a wearer on a foot received within footwear 510, first edge 570 may be substantially aligned with second edge 572. In addition, first indicia portion 536 may include a third edge 574 and second indicia portion 540 may include a fourth edge 576. When sock 538 is worn by a wearer on a foot received within footwear 510, third edge 574 and fourth edge 576 may be arranged substantially facing each other. It will be noted that other shapes and configurations for indicia 534 are also possible.

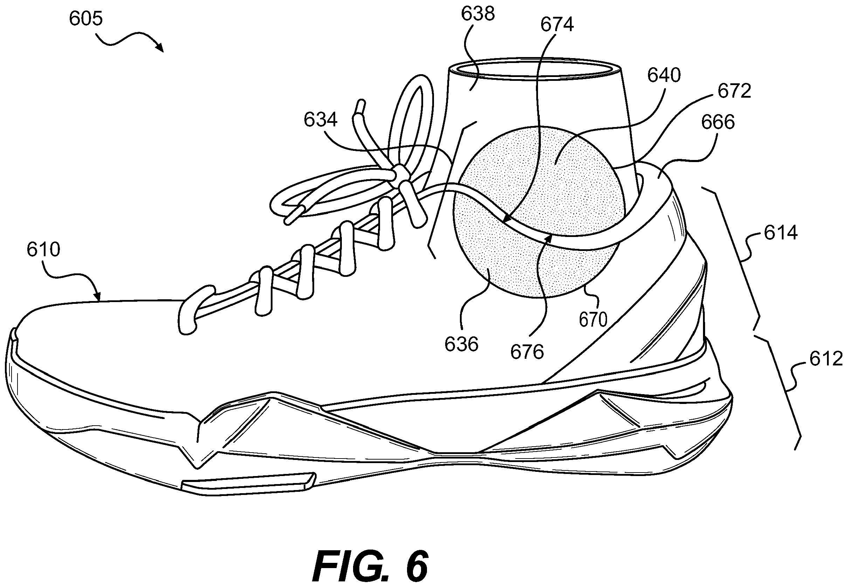

In some embodiments, the indicia may have other shapes. For example, as shown in FIG. 6, a footwear system 605 may include an article of footwear 610 having a sole structure 612 secured to an upper 614. Upper 614 may define an opening 622 configured to receive the foot of a wearer. System 605 may also include a sock 638. When combined, markings on footwear 610 and sock 638 may for an indicia 634 formed by indicia portions on both footwear 610 and sock 638, as shown in FIG. 6. For example, upper 614 may include a first indicia portion 636, at least a portion of which may be disposed adjacent opening 622. Sock 638 may include a second indicia portion 640. When sock 638 is disposed on the foot of a wearer received within footwear 610, first indicia portion 636 may substantially align with second indicia 640 to form indicia 634. Further, as shown in FIG. 6, a liner 666 may create discontinuity between first indicia portion 636 and second indicia portion 640.

First indicia portion 636 may include a first edge 670 and second indicia portion 640 may include a second edge 672. When sock 638 is worn by a wearer on a foot received within footwear 610, first edge 670 may be substantially aligned with second edge 672. In addition, first indicia portion 636 may include a third edge 674 and second indicia portion 640 may include a fourth edge 676. When sock 638 is worn by a wearer on a foot received within footwear 610, third edge 674 and fourth edge 676 may be arranged substantially facing each other. It will be noted that other shapes and configurations for indicia 634 are also possible.

In some embodiments, the sock may include a proprioceptive component. For example, in some embodiments, the sock may include a cuff or strap configured to wrap around the ankle portion of the sock. Such a strap may be fastenable around the ankle to provide improved tactile feedback regarding the positioning of the foot and the loading of the shoe against the foot. Because the strap may be made out of cloth, textile, or other lightweight material, the proprioceptive benefit may be provided without the extra weight and/or restriction of movement that may result from simply making the upper of the shoe higher on the ankle. In some embodiments, the strap may provide a thermal benefit by keeping the ankle warm, which may promote flexibility and potentially prevent injury. Further, in some embodiments, the strap may provide compression, which may promote blood circulation and/or reduce/prevent swelling in the ankle and foot.

In embodiments in which the sock includes a strap, the indicia may be incorporated on to portions of the strap. By incorporating the indicia onto the strap, both the enlarged indicia and proprioceptive benefits may be implemented in the same footwear system.

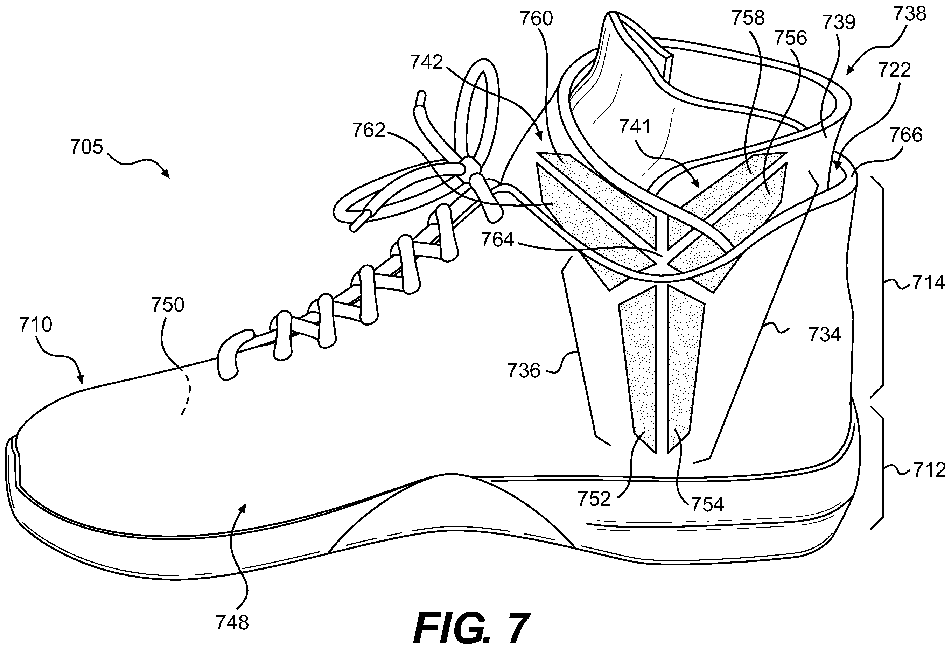

FIGS. 7-9 illustrate a footwear system 705, which may include an article of footwear 710 having a sole structure 712 secured to an upper 714. Upper 714 may define an opening 722 configured to receive the foot of a wearer. System 705 may also include a sock 738. As shown in FIG. 7, system 705 may include an indicia 734, which may be formed by indicia portions disposed on footwear 710 and sock 738. For example, the external surface of upper 714 of footwear 710 may include a first indicia portion 736, at least a portion of which may be disposed adjacent opening 722, as shown in FIG. 7. In some embodiments, first indicia portion 736 may be disposed on a medial side 748 of footwear 710, as shown in FIG. 7. In some embodiments, first indicia portion 736 may be disposed on a lateral side 750 of footwear 710 In addition, sock 738 may include a second indicia portion 741 and a third indicia portion 742. As shown in FIG. 7, when sock 738 is worn on a foot received within footwear 710, first indicia portion 738, second indicia portion 741, and third indicia portion 742 may be substantially aligned to form indicia 734.

Sock 738 may include a base portion 739 and a strap portion 740 configured to wrap at least partially around an ankle region of base portion 739 of sock 738. Second indicia portion 741 may be disposed on base portion 739 of sock 738 and third indicia portion 742 may be disposed on strap portion 740.

In some embodiments, indicia 734 may include three pair of elongate markings, wherein each pair of elongate markings includes a first marking and a second marking oriented substantially parallel to the first marking, each pair of elongate markings extending substantially radially from a midpoint. For example, indicia 734 may include a first marking 752 and a second marking 754, which may be substantially parallel to one another. In addition, indicia 734 may also include a third marking 756 and a fourth marking 758, which may be substantially parallel to one another, and a fifth marking 760 and a sixth marking 762, which may also be substantially parallel to one another. Each pair of markings may extend substantially radially from a central region 764.

The markings of indicia 734 may extend across a single or multiple components of system 705. For example, as shown in FIG. 7, first marking 752 and second marking 754 are completely disposed on footwear 710. However, third marking 756 and sixth marking 762 extend across both sock 738 and footwear 710. In addition, third marking 756 and fourth marking 758 extend across both base portion 739 and strap portion 740 of sock 738. In some embodiments, however, discrete markings of an indicia may be strictly disposed on a single component. In some embodiments, a liner 766 may be visible proximate opening 722 and may interrupt indicia 734 or may be colored to integrate with indicia 734 as discussed above regarding other disclosed embodiments.

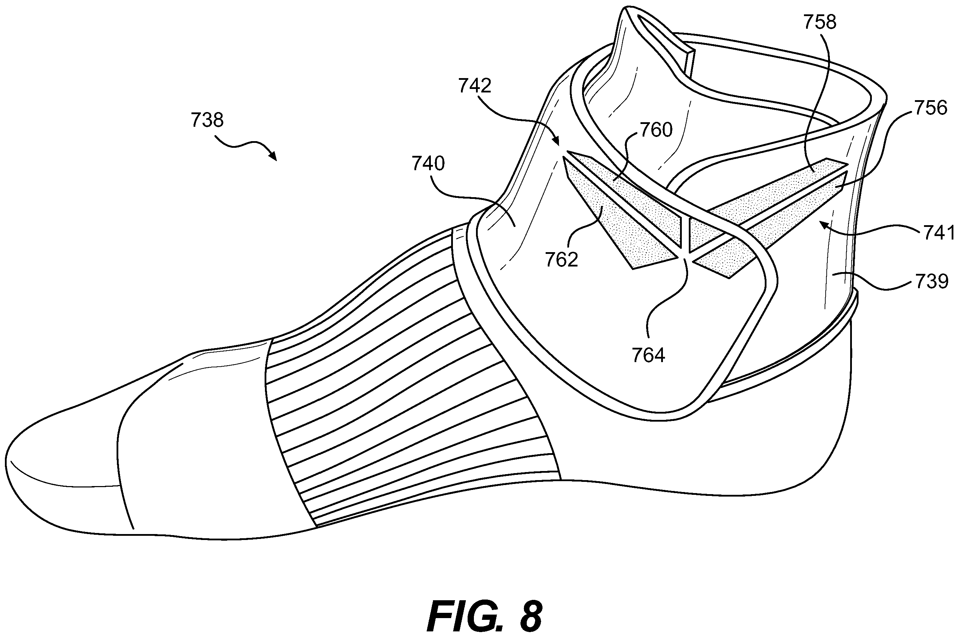

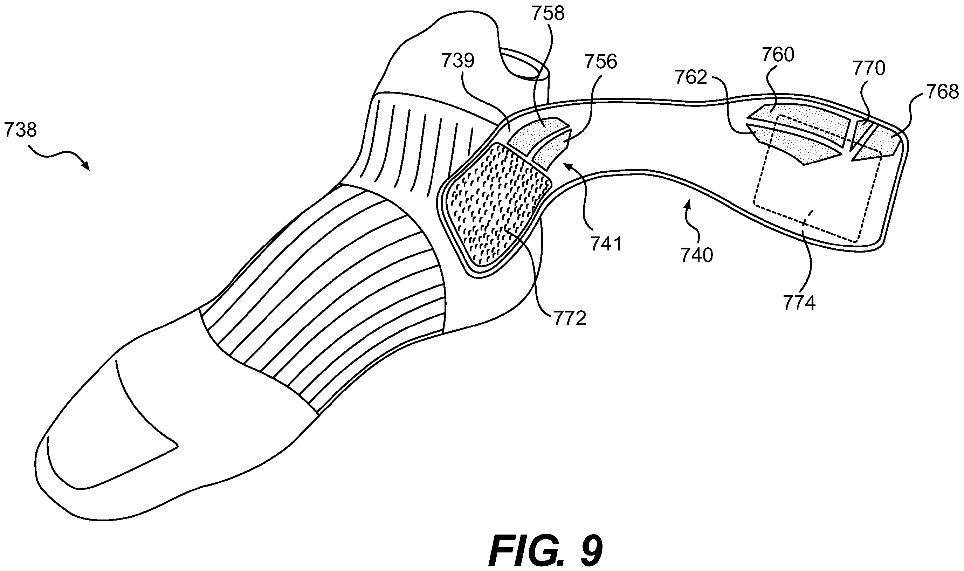

FIGS. 8 and 9 illustrate sock 738 by itself without footwear 710. FIG. 8 shows strap portion 740 in a fastened condition, as it would be when sock 738 is worn within footwear 710. As illustrated in FIG. 8, in some embodiments, third marking 756 and sixth marking 762 may be fully represented on sock 738, even though small portions of third marking 756 and sixth marking 762 are also represented on upper 714 (see FIG. 7). This may ensure that there is desired continuity between the portions of indicia 734 on sock 738 and the portions of indicia 734 on footwear 710, as sock 738 may sit at different heights on the ankle depending on the shape and size of the foot of the wearer, how tightly the wearer pulls the sock onto the foot, and the positioning of strap portion 740 when the wearer fastens it around the ankle.

FIG. 9 illustrates sock 738 with strap portion 740 unfastened. As shown in FIG. 9, a second portion 768 of third marking 756 may be provided on the distal end of strap portion 740. Similarly, a second portion 770 of fourth marking 758 may also be provided on the distal end of strap portion 740. As also shown in FIG. 9, sock 738 may include fastening portions for fastening strap 740 against base portion 739 of sock 738. For example, a first fastening portion 772 may be provided on base portion 739 and a second fastening portion 774 may be provided at the distal end of strap portion 740, as shown in FIG. 9. First fastening portion 772 and second fastening portion 774 may, for example, include hook and loop fastening patches. In some embodiments, however, alternative fastening structures may be implemented.

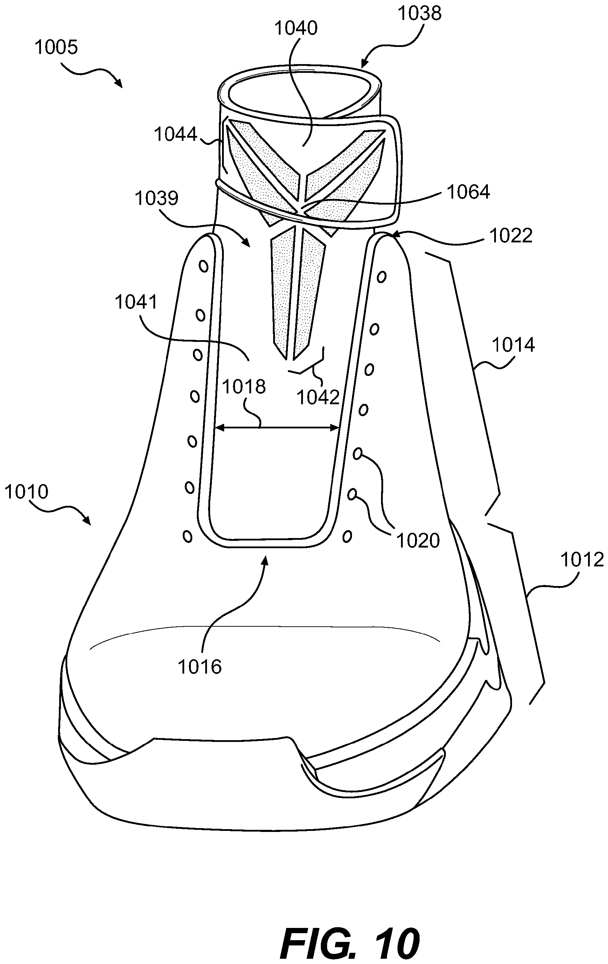

FIG. 10 illustrates a footwear system 1005, which may include an article of footwear 1010 having a sole structure 1012 secured to an upper 1014. Upper 1014 may define an opening 1022 configured to receive the foot of a wearer. System 1005 may also include a sock 1038.

As shown in FIG. 10, footwear 1010 may include an instep region 1016. In addition, opening 1022 may be continuous with a lacing gap 1018, which may extend along instep region 1016 of footwear 1010. Lacing gap 1018 may be disposed, for example, between portions of upper 1014 including lace eyelets 1020. In some embodiments, footwear 1010 may be provided without a tongue in lacing gap 1018, and an instep portion 1041 may serve as the tongue of footwear 1010 to protect the foot of the wearer from laces (not shown) threaded through eyelets 1020.

In some embodiments, sock 1038 may include indicia that is visible in lacing gap 1018. Further, in some embodiments, the indicia may be formed by the combination of indicia portions on a base portion of the sock and a strap portion of the sock. For example, as shown in FIG. 10, sock 1038 may include a base portion 1039, and a strap portion 1040 which may wrap at least partially around an ankle region of base portion 1039. A discussion of the advantages of such a strap portion is provided above regarding other embodiments.

Accordingly, as shown in FIG. 10, instep portion 1041 of sock 1038 may be configured to be externally exposed through lacing gap 1018 of footwear 1010 when sock 1038 is worn by the wearer on a foot received within upper 1014. In some embodiments, sock 1038 may also include a first indicia portion 1042 disposed on base portion 1039 of sock 1038 and a second indicia portion 1044 disposed on strap portion 1040 of sock 1038. As shown in FIG. 10, when sock 1038 is worn by the wearer on a foot received within upper 1014, first indicia portion 1042 and second indicia portion 1044 are substantially aligned to form an indicia in instep region 1041 of sock 1038, the indicia being exposed through lacing gap 1018 of footwear 1010. As further shown in FIG. 10, in some embodiments, the indicia may include three pair of elongate markings, wherein each pair of elongate markings includes a first marking and a second marking oriented substantially parallel to the first marking, each pair of elongate markings extending substantially radially from a midpoint 1064.

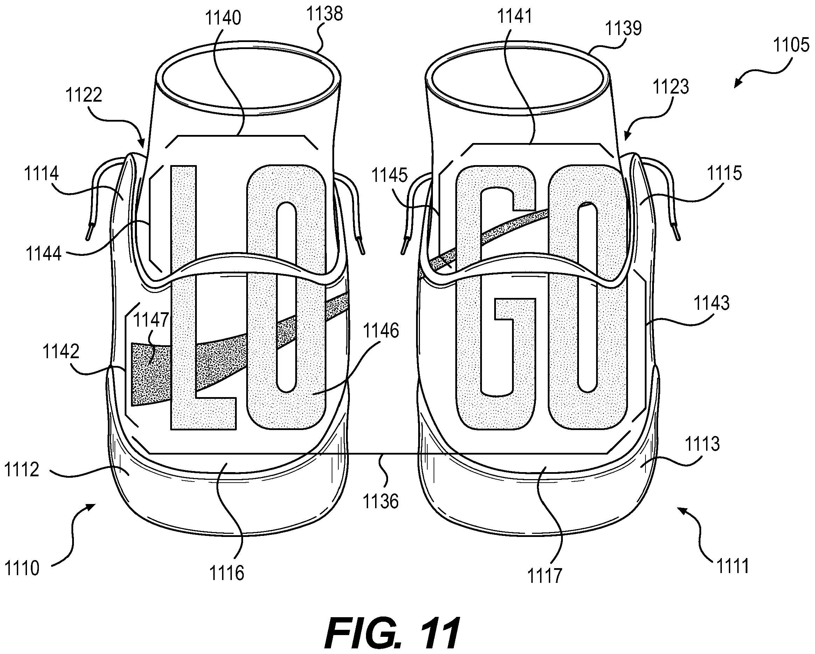

FIG. 11 shows a rear view of a footwear system 1105 including a pair of footwear and a pair of socks. For example, system 1105 may include a first article of footwear 1110 having a first sole structure 1112 secured to a first upper 1114. First upper 1114 may define a first opening 1122 configured to receive the foot of a wearer. System 1105 may also include a first sock 1138. First footwear 1110 and first sock 1138 may be configured, for example, to accommodate a left foot of a wearer. In addition, system 1105 may also include a second article of footwear 1111 having a second sole structure 1113 secured to a second upper 1115. Second upper 1115 may define a second opening 1123 configured to receive the foot of a wearer. System 1105 may also include a second sock 1139. Second footwear 1111 and second sock 1139 may be configured, for example, to accommodate a right foot of a wearer.

As shown in FIG. 11, system 1105 may include an indicia 1136 which may be formed by the combination of indicia portions disposed on first footwear 1110, second footwear 1111, first sock 1138, and second sock 1139. A left indicia portion 1140 may be formed by the combination of indicia portions on a first heel region 1116 of first footwear 1110 and first sock 1138, and a right indicia portion 1141 may be formed by the combination of indicia portions on a second heel region 1117 of second footwear 1111 and second sock 1139.

As shown in FIG. 11, first sock 1138 may include a first section 1144 of indicia 1136 and right sock 1139 may include a second section 1145 of indicia 1136. In some embodiments, first section 1144 of indicia 1136 may be different than second section 1145 of indicia 1136. However, in some embodiments, first section 1144 and second section 1145 may be the same. In some embodiments, when first sock 1138 and right sock 1139 are worn by a wearer, first section 1144 of indicia 1136 and second section 1145 of indicia 1136 substantially align with one another to form a subportion of indicia 1136.

As also shown in FIG. 11, first footwear 1110 may include a third section 1142 of indicia 1136 and second footwear 1111 may include a fourth section 1143 of indicia 1136. In some embodiments, third section 1142 of indicia 1136 may be different than fourth section 1143 of indicia 1136, as shown in FIG. 11. As further shown in FIG. 11, when first footwear 1110, second footwear 1111, first sock 1138, and second sock 1139 are worn together, first section 1144, second section 1145, third section 1142, and fourth section 1143 of indicia 1136 may substantially align with one another to form indicia 1136.

As shown in FIG. 11, indicia 1136 or its various subcomponents may include any desired graphical configuration. For example, in some embodiments, all or a portion of indicia 1136 may include alpha-numeric indicia 1146, as shown in FIG. 11. Alternatively or additionally, indicia 1136 may include a graphical shape, such as a stripe 1147, as shown in FIG. 11. Other types of graphics are also possible. It should also be noted that the various portions of indicia 1136 may have varying colors and/or other visual properties, such as reflectivity, shine, sparkle. The indicia portions on the left shoe and/or sock may be the same or different than those on the right shoe and/or sock. As shown in FIG. 11, when the indicia portions on the left shoe and/or sock are different than those on the right shoe and/or sock, features of the left and right indicia portions may align with one another when the shoes are situated next to each other to form a larger indicia having components on both the left and right shoe/sock combinations.

In some embodiments, a footwear system may include multiple socks that are interchangeably wearable with the same article of footwear. Each sock may have a different indicia portion that combines with an indicia portion on the same article of footwear to form different indicia. For example, the footwear may have a first indicia portion, a first sock may have a second indicia portion, and a second sock may have a third indicia portion. When the article of footwear is worn with the first sock, the first indicia portion on the footwear and the second indicia portion on the first sock may substantially align with one another to form a first indicia. When the article of footwear is worn with the second sock, the third indicia portion on the second sock substantially aligns with one or more features of the first indicia portion on the upper of the footwear to form a second indicia that is different from the first indicia formed by the combination of the first sock with the article of footwear. In order to provide this interchangeability, the first indicia portion on the article of footwear may form a portion of a larger indicia, such that the indicia portion is common to all of the different indicia formed by the different interchangeable socks.

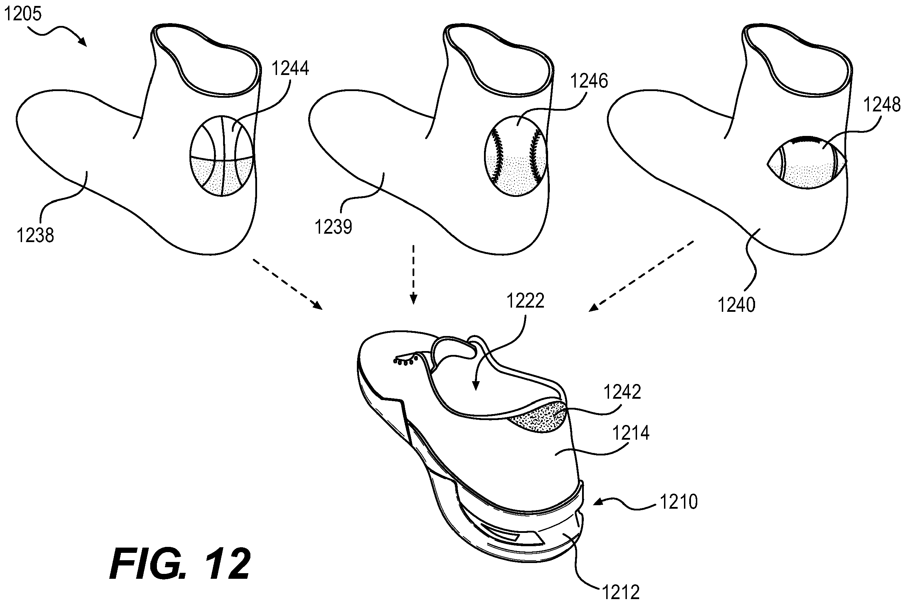

As illustrated in FIG. 12, a footwear system 1205 may include an article of footwear 1210, which may include a sole structure 1212 and an upper 1214 secured to sole structure 1212. Upper 1214 may define an opening 1222. Adjacent opening 1222, upper 1214 may include a first indicia portion 1242. In FIG. 12, an exemplary first indicia portion 1242 is shown as a shaded shape having a curved lower portion. However, other shapes/graphics may be used as desired.

As also illustrated in FIG. 12, system 1205 may include two or more interchangeable socks. For example, system 1205 may include a first sock 1238, a second sock 1239, and a third sock 1240. First sock 1238 may include a second indicia portion 1244, second sock 1239 may include a third indicia portion 1246, and third sock 1240 may include a fourth indicia portion 1248. As shown in FIG. 12, second indicia portion 1244 may be a basketball, for example, third indicia portion 1246 may include a baseball, and fourth indicia portion 1248 may include a football. In some embodiments, the indicia portions on the socks may have the same general shape, but may be differently colored in order to form a different pattern. For example, second indicia portion 1244 and third indicia portion 1246 both are shown as having round shapes, however, the coloration of these two indicia portions are different such that one looks like a basketball and the other looks like a baseball. In other embodiments, the indicia portions on the sock may have different shapes that both align with first indicia portion 1242. For example, third indicia portion 1246 is shown as having a round shape and fourth indicia portion 1248 is shown as having an oblong (football) shape. However, the lower curved portion of both the round shape and the oblong shape may each align with the rounded shape of first indicia portion 1242.

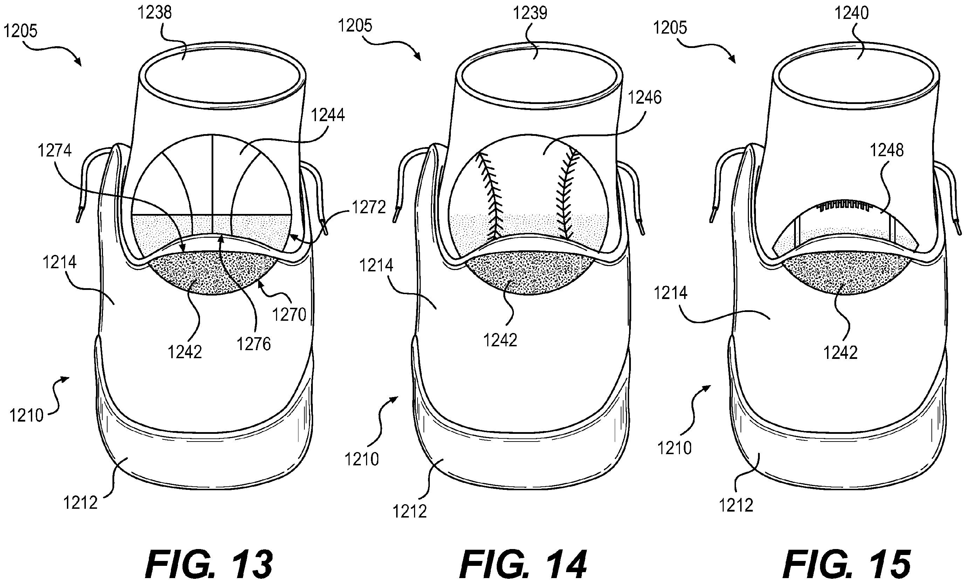

FIGS. 13-15 show the combinations of each of the interchangeable socks with footwear 1210 to illustrate the differing effects. While FIGS. 13 and 14 illustrate how round indicia portions may align with first indicia portion 1242 on footwear 1210, FIG. 15 shows how an oblong shape like a football may be similarly completed by first indicia portion 1242 because of the curved lower edge of first indicia portion 1242. These graphics are shown to illustrate the interchangeability of socks generally. Accordingly, other graphics may be used in a similar interchangeable way.

First indicia portion 1242 may include a first edge 1270 and second indicia portion 1244 may include a second edge 1272. When sock 1238 is worn by a wearer on a foot received within footwear 1210, first edge 1270 may be substantially aligned with second edge 1272. In addition, first indicia portion 1242 may include a third edge 1274 and second indicia portion 1244 may include a fourth edge 1276. When sock 1238 is worn by a wearer on a foot received within footwear 1210, third edge 1274 and fourth edge 1276 may be arranged substantially facing each other.

It will also be noted that the coloration of the indicia portions may be varied to further contribute to the continuity between the indicia portions on the socks and the indicia portion on the footwear. For example, as shown in FIGS. 13-15, first indicia portion may have a darker color than the indicia portions on the socks. Further, the lower portions of the indicia portions on the socks may also be darker than the upper portions of the indicia portions on the socks. The darkness may appear as a shadow on a curved underside of the representations of sports balls. By providing first indicia portion 1242 in a relatively dark color, the lower portion of the larger indicia formed by the combination may simply appear as a heavily shadowed surface. Accordingly, first indicia portion 1242 may omit any of the distinctive markings of each type of sports ball, such as the seams on the basketball and baseball, and the stripes on the football, thus providing first indicia portion 1242 as a generic portion of a ball shape. While the embodiment shown in FIGS. 12-15 shows interchangeable socks, in some embodiments, a footwear system may include multiple articles of footwear having differing indicia portions, the multiple articles of footwear being interchangeably wearable with the same sock in order to form differing indicia.

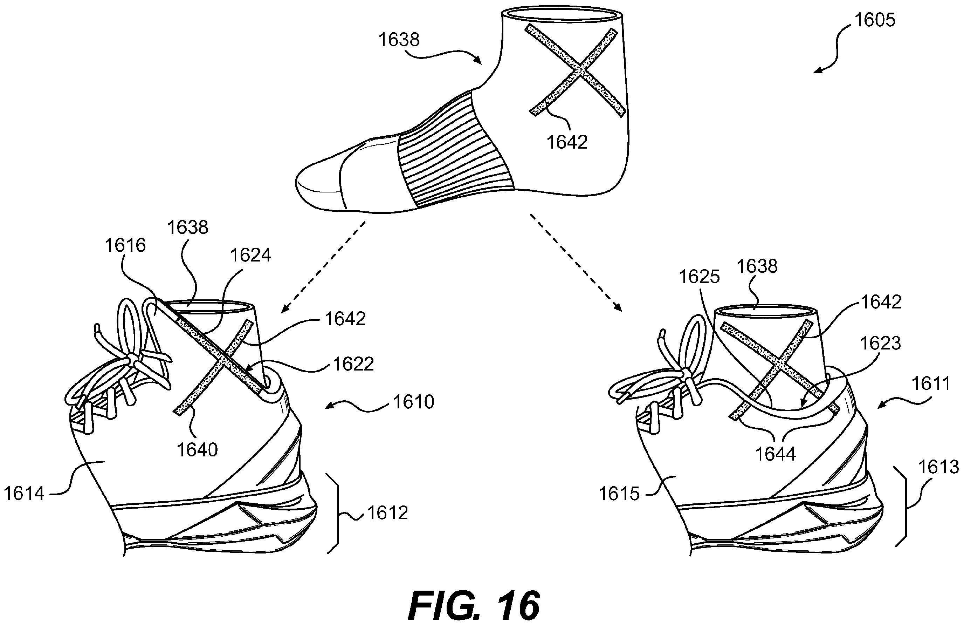

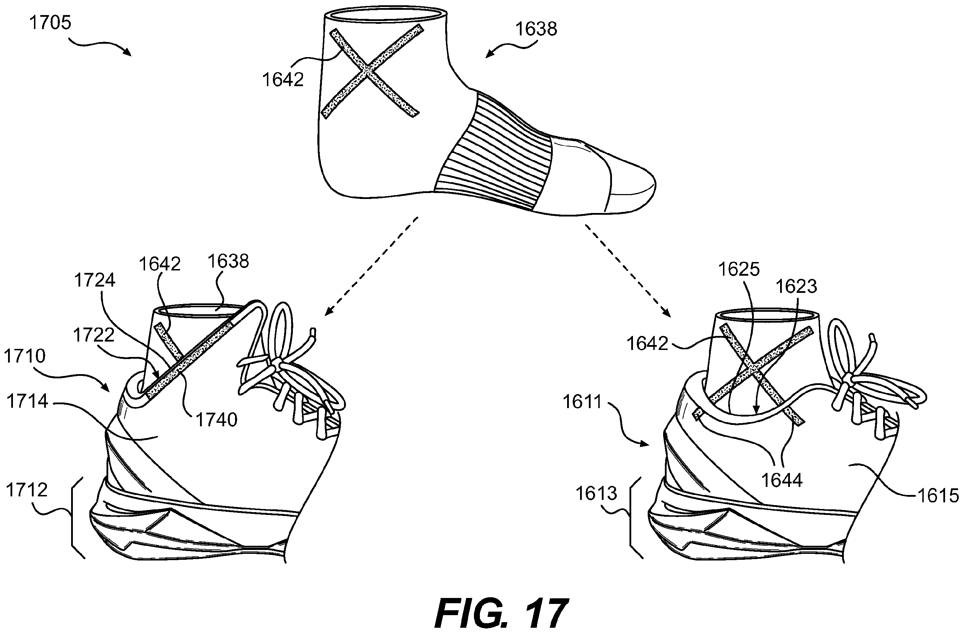

In some systems the openings in the upper of interchangeable footwear may be different. Thus the opening of one article of footwear may be configured to expose a different amount of an indicia portion on a sock than the opening of a second article of footwear. As shown in FIGS. 16 and 17, the same sock may be used with differently configured footwear to form an indicia. For example, FIG. 16 shows a footwear system 1605, which may include a sock 1638, a first article of footwear 1610, and a second article of footwear 1611. First footwear 1610 may include a first sole structure 1612 secured to a first upper 1614. First upper 1614 may define a first opening 1622 configured to receive a foot of a wearer. Second footwear 1611 may include a second sole structure 1613 secured to second upper 1615. Second upper 1614 may define a second opening 1623.

First upper 1614 of first footwear 1610 may have a different configuration than second upper 1615 of second footwear 1611. For example, first footwear 1610 may have a higher extending first upper 1614 than second upper 1615. For instance, first upper 1614 may extend upward to an upper portion 1616, whereas second upper 1615 may be more of a low-cut configuration.

First upper 1614 may include a first indicia portion 1640 adjacent first Opening 1622. In addition, sock 1638 may include a second indicia portion 1642. Second indicia portion 1642 may have any suitable configuration, such as an "X," as shown in FIG. 16. Further, when sock 1638 is worn on the foot of a wearer that is received within first upper 1614 of first footwear 1610, second indicia portion 1642 may substantially align with first indicia portion 1640 to form an indicia. In the embodiment shown in FIG. 16, the indicia formed by the sock/footwear combination may be an "X."

Second upper 1615 may include a third indicia 1644 adjacent to second opening 1623. Third indicia portion 1644 may be configured to align with second indicia portion 1642 on sock 1638 when sock 1638 and second footwear 1611 are worn by the wearer. Because a second edge 1625 of second upper 1615 defining second opening 1623 extends less distance upward than a first edge 1624 of first upper 1614 defining first opening 1622, more of second indicia 1642 on sock 1638 may be exposed when sock 1638 is worn with second footwear 1611 than when worn with first footwear 1610. As shown in FIG. 16, however, despite this difference in the configuration of first upper 1614 and second upper 1615, the indicia formed by the combination of second indicia portion 1642 with third indicia portion 1644 may be substantially the same as the indicia formed by the combination of second indicia portion 1642 with first indicia portion 1640.

In some embodiments, interchangeable articles of footwear may be differently configured and, when combined with a common sock, may form different indicia. FIG. 17 shows a system 1705, which includes the same sock 1638 and second footwear 1611 shown in FIG. 16, and further includes a third article of footwear 1710. Third footwear 1710 may include a third sole structure 1712 secured to a third upper 1714, which defines an opening 1722. Third upper 1714 may include a fourth indicia portion 1740 adjacent an edge 1724 defining opening 1722.

Fourth indicia portion 1740 may be configured such that, when combined with first sock 1638, a different indicia is formed than when second footwear 1611 is combined with sock 1638. For example, when sock 1638 is worn by the wearer on a foot received within second upper 1614 of second footwear 1611, third indicia portion 1644 aligns with second indicia portion 1642 to form a first indicia. When sock 1638 is worn by the wearer on a foot received within third upper 1714 of third footwear 1710, fourth indicia portion 1740 may substantially align with second indicia portion 1642 on sock 1638 to form a second indicia that is different than the first indicia. For example, because edge 1724 may be angled differently and may extend higher than edge 1625, upper 1714 may cover more of second indicia portion 1642 on sock 1638. Therefore, as shown in FIG. 17, the second indicia may be formed as a "Y" instead of the "X" formed by the first indicia.

It will be understood that the configuration, alignment, and coloration of the indicia and indicia portions may vary. Further, socks and/or footwear having indicia portions may be interchangeable to enable a customizable appearance of the sock/footwear combination. Indicia and indicia portions may be monochromatic or polychromatic.

In addition to the coloration of the indicia portions, the coloration of other portions of the footwear upper and sock may have varying coloration that provides a background for the indicia. In some cases, the sock and footwear upper may have the same or similar background coloration. In other cases, the sock and footwear upper may have differing background coloration.

## Claims

1. A footwear system (105), comprising:
a first article of footwear (110) including a first sole structure (112) and a first upper (114) secured to the first sole structure (112), the first article of footwear (110) having a forefoot region (116), a midfoot region (118) and a heel region (120), the first upper (114) defining a first opening (122) configured to receive a foot of a wearer, wherein the first upper (114) includes a first indicia portion (136) disposed adjacent to the first opening (122); and
a sock including a second indicia portion (140), wherein, one or more features of said second indicia portion (140) are suitable to be substantially aligned with one or more features of the first indicia portion (136) on the upper to form a first indicia (134) when the sock (138) is worn by the wearer on a foot received within the upper (114);
wherein the first indicia portion (136) includes a first pair of elongate markings (142) disposed on a rear of a heel region of the upper (114) and extending in a substantially vertical direction;
wherein the second indicia portion (140) disposed on the sock (138) includes a second pair of elongate markings (144) extending upward and toward a medial side (148) of the sock (138), and a third pair of elongate markings (146) extending upward and toward a lateral side (150) of the sock (138);
wherein the first pair of elongate markings (142) includes a first marking (152) and a second marking (154) oriented substantially parallel to the first marking (152);
wherein the second pair of elongate markings (144) includes a third marking (156) and a fourth marking (158) oriented substantially parallel to the third marking (156);
wherein the third pair of elongate markings (146) includes a fifth marking (160) and a six marking (162) oriented substantially parallel to the fifth marking (160);
wherein each pair of elongate markings (142, 144, 146) are configured to extend substantially radially from a central region (165);
wherein the first marking (152) includes a first inner edge (170);
wherein the second marking (154) includes a second inner edge (172);
wherein the first inner edge (170) is substantially parallel to the second inner edge (172);
wherein the first inner edge (170) of the first marking (152) is in substantial alignment with a third edge (178) of fifth marking (160);
wherein the second inner edge (172) of the second marking (154) is in substantial alignment with a fourth edge (180) of the third marking (156); and
wherein the sock (138) includes a forefoot region, a midfoot region and a heel region.

2. The system (105) of claim 1, wherein, when the sock (138) is worn by the wearer on a foot received within the upper (114), one or more features of the second indicia portion (140) are in substantial non-alignment with one or more features of the first indicia portion (136) on the upper (114).

3. The system of claim 1, wherein the system (1105) includes a pair of footwear including a left footwear (1110) configured to receive a left foot of the wearer and a right footwear (1111) configured to receive the right foot of the wearer, the system (1105) further including a pair of socks including a left sock (1138) configured to receive a left foot of the wearer and a right sock (1139) configured to receive the right foot of the wearer, wherein the left sock (1138) includes a first section (1144) of the first indicia (1136) and the right sock (1139) includes a second section (1145) of the first indicia (1136), and wherein the first section (1144) of the first indicia (1136) is different than the second section (1145) of the first indicia (1136).

4. The system of claim 3, wherein, when the left sock (1138) and the right sock (1139) are worn by the wearer, the first section (1144) of the first indicia (1136) and the second section (1145) of the first indicia (1136) substantially align with one another to form a subportion of the first indicia (1136).

5. The system of claim 1, wherein the system (1105) includes a pair of footwear including a left footwear (1110) configured to receive a left foot of the wearer and a right footwear (1111) configured to receive the right foot of the wearer, the system (1105) further including a left sock (1138) configured to receive a left foot of the wearer and a right sock (1139) configured to receive the right foot of the wearer;
wherein the left sock (1138) includes a first section (1144) of the first indicia (1136) and the right sock (1139) includes a second section (1145) of the first indicia (1136); and
wherein the left footwear (1110) includes a third section (1142) of the first indicia (1136) and the right footwear (1111) includes a fourth section (1143) of the first indicia (1136), and wherein the third section (1142) of the first indicia (1136) is different than the fourth section (1143) of the first indicia (1136).

6. The system of claim 5, wherein the first section (1144) of the first indicia (1136), the second section (1145) of the first indicia (1136), the third section (1142) of the first indicia (1136), and the fourth section (1143) of the first indicia (1136) substantially align with one another to form the first indicia (1136).

7. The system of claim 1, wherein the sock is a first sock (1238) and the system (1205) further includes a second sock (1239, 1240) that is interchangeable with the first sock (1238);
wherein the second sock (1239, 1240) includes a third indicia portion (1246, 1248); and
wherein, when the second sock (1239, 1240) is worn by the wearer on a foot received within the first upper (1214), the third indicia portion (1246, 1248) substantially aligns with one or more features of the first indicia portion (1242) on the first upper (1214) to form a second indicia that is different from the first indicia.

8. The system of claim 1, wherein the system further includes a second article of footwear (1611; 1710) that is interchangeable with the first article of footwear (1610);
the second article of footwear (1611; 1710) including a second sole structure (1613; 1712) and a second upper (1614; 1714) secured to the second sole structure (1613; 1712), the second upper (1615; 1714) defining a second opening (1623; 1722) configured to receive a foot of a wearer, wherein the second upper (1615; 1714) includes a third indicia portion (1644; 1740) disposed adjacent to the second opening (1623; 1722), the third indicia portion (1644; 1740) configured to align with the second indicia portion (1642) on the sock (1638) when the sock (1638) and the second article of footwear (1611; 1710) are worn by the wearer; and
wherein the second opening (1623; 1722) of the second article of footwear (1611; 1710) is configured to expose a different amount of the second indicia portion (1642) when worn by the wearer than the first article of footwear (1610).

9. The system of claim 8, wherein the third indicia portion is substantially the same as the first indicia portion (136); and
wherein, when the sock (1638) is worn by the wearer on a foot received within the second upper (1714) of the second article of footwear (1710), the third indicia portion (1740) aligns with the second indicia portion (1642) to form a second indicia that is different than the first indicia.

## Patentansprüche

1. Schuhsystem (105), das Folgendes umfasst:
einen ersten Schuhartikel (110), der eine erste Sohlenstruktur (112) und ein erstes Oberteil (114) umfasst, das an der ersten Sohlenstruktur (112) gesichert ist, wobei der erste Schuhartikel (110) eine Vorfußregion (116), eine Mittelfußregion (119) und eine Fersenregion (120) aufweist, wobei das erste Oberteil (114) eine erste Öffnung (122) bestimmt, die ausgestaltet ist, einen Fuß eines Trägers aufzunehmen, wobei das erste Oberteil (114) einen ersten Markierungsabschnitt (136) umfasst, der benachbart zur ersten Öffnung (112) angeordnet ist; und
eine Socke, die einen zweiten Markierungsabschnitt (140) umfasst, wobei ein oder mehrere Merkmale des zweiten Markierungsabschnitts (140) geeignet sind, im Wesentlichen mit einem oder mehreren Merkmalen des ersten Markierungsabschnitts (136) auf dem ersten Oberteil ausgerichtet zu sein, um eine erste Markierung (134) zu bilden, wenn die Socke (138) vom Träger an einem Fuß getragen wird, der im Oberteil (114) aufgenommen wird;
wobei der erste Markierungsabschnitt (136) ein erstes Paar länglicher Kennzeichnungen (142) umfasst, die auf einer Rückseite einer Fersenregion des Oberteils (114) angeordnet sind und sich in einer im Wesentlichen vertikalen Richtung erstrecken;
wobei der zweite Markierungsabschnitt (140), der auf der Socke (138) angeordnet ist, ein zweites Paar länglicher Kennzeichnungen (144), die sich nach oben und zu einer medialen Seite (148) der Socke (138) erstrecken, und ein drittes Paar länglicher Kennzeichnungen (146) umfasst, die sich nach oben und zu einer lateralen Seite (150) der Socke (138) erstrecken;
wobei das erste Paar länglicher Kennzeichnungen (142) eine erste Kennzeichnung (152) und eine zweite Kennzeichnung (154) umfasst, die im Wesentlichen parallel zur ersten Kennzeichnung (152) ausgerichtet ist;
wobei das zweite Paar länglicher Kennzeichnungen (144) eine dritte Kennzeichnung (156) und eine vierte Kennzeichnung (158) umfasst, die im Wesentlichen parallel zur dritten Kennzeichnung (156) ausgerichtet ist;
wobei das dritte Paar länglicher Kennzeichnungen (146) eine fünfte Kennzeichnung (160) und eine sechste Kennzeichnung (162) umfasst, die im Wesentlichen parallel zur fünften Kennzeichnung (160) ausgerichtet ist;
wobei jedes Paar länglicher Kennzeichnungen (142, 144, 146) ausgestaltet ist, sich im Wesentlichen radial von einer mittleren Region (165) zu erstrecken;
wobei die erste Kennzeichnung (152) einen ersten inneren Rand (170) umfasst;
wobei die zweite Kennzeichnung (154) einen zweiten inneren Rand (172) umfasst;
wobei der erste innere Rand (170) im Wesentlichen parallel zum zweiten inneren Rand (172) ist;
wobei der erste innere Rand (170) der ersten Kennzeichnung (152) im Wesentlichen mit einem dritten Rand (178) der fünften Kennzeichnung (160) ausgerichtet ist;
wobei der zweite innere Rand (172) der zweiten Kennzeichnung (154) im Wesentlichen mit einem vierten Rand (180) der dritten Kennzeichnung (156) ausgerichtet ist; und
wobei die Socke (138) eine Vorfußregion, eine Mittelfußregion und eine Fersenregion umfasst.

2. System (105) nach Anspruch 1, wobei, wenn die Socke (138) vom Träger an einem Fuß getragen wird, der im Oberteil (114) aufgenommen wird, ein oder mehrere Merkmale des zweiten Markierungsabschnitts (140) im Wesentlichen nicht mit einem oder mehreren Merkmalen des ersten Markierungsabschnitts (136) auf dem Oberteil (114) ausgerichtet sind.

3. System nach Anspruch 1, wobei das System (1105) ein Paar Schuhe umfasst, das einen linken Schuh (1110), der ausgestaltet ist, einen linken Fuß des Trägers aufzunehmen, und einen rechten Schuh (1111), der ausgestaltet ist, den rechten Fuß des Trägers aufzunehmen, umfasst, wobei das System (1105) überdies ein Paar Socken umfasst, das eine linke Socke (1138), die ausgestaltet ist, einen linken Fuß des Trägers aufzunehmen, und eine rechte Socke (1139), die ausgestaltet ist, den rechen Fuß des Trägers aufzunehmen, umfasst, wobei die linke Socke (1138) einen ersten Bereich (1144) der ersten Markierung (1136) umfasst und die rechte Socke (1139) einen zweiten Bereich (1145) der ersten Markierung (1136) umfasst, und wobei der erste Bereich (1144) der ersten Markierung (1136) sich von dem zweiten Bereich (1145) der ersten Markierung (1136) unterscheidet.

4. System nach Anspruch 3, wobei, wenn die linke Socke (1138) und die rechte Socke (1139) vom Träger getragen werden, der erste Bereich (1144) der ersten Markierung (1136) und der zweite Bereich (1145) der ersten Markierung (1136) im Wesentlichen miteinander ausgerichtet sind, um einen Unterabschnitt der ersten Markierung (1136) zu bilden.

5. System nach Anspruch 1, wobei das System (1105) ein Paar Schuhe umfasst, das einen linken Schuh (1110), der ausgestaltet ist, einen linken Fuß des Trägers aufzunehmen, und einen rechten Schuh (1111), der ausgestaltet ist, den rechten Fuß des Trägers aufzunehmen, umfasst, wobei das System (1105) überdies eine linke Socke (1138), die ausgestaltet ist, einen linken Fuß des Trägers aufzunehmen, und eine rechte Socke (1139), die ausgestaltet ist, den rechten Fuß des Trägers aufzunehmen, umfasst;
wobei die linke Socke (1138) einen ersten Bereich (1144) der ersten Markierung (1136) umfasst und die rechte Socke einen zweiten Bereich (1145) der ersten Markierung (1136) umfasst; und
wobei der linke Schuh (1110) einen dritten Bereich (1142) der ersten Markierung (1136) umfasst und der rechte Schuh (1111) einen vierten Bereich (1143) der ersten Markierung (1136) umfasst, und wobei der dritte Bereich (1142) der ersten Markierung (1136) sich von dem vierten Bereich (1143) der ersten Markierung (1136) unterscheidet.

6. System nach Anspruch 5, wobei der erste Bereich (1144) der ersten Markierung (1136), der zweite Bereich (1145) der ersten Markierung (1136), der dritte Bereich (1142) der ersten Markierung (1136) und der vierte Bereich (1143) der ersten Markierung (1136) im Wesentlichen miteinander ausgerichtet sind, um die erste Markierung (1136) zu bilden.

7. System nach Anspruch 1, wobei die Socke eine erste Socke (1238) ist und das System (1205) überdies eine zweite Socke (1239, 1240) umfasst, die mit der ersten Socke (1238) austauschbar ist;
wobei die zweite Socke (1239, 1240) einen dritten Markierungsabschnitt (1246, 1248) umfasst; und
wobei, wenn die zweite Socke (1239, 1240) vom Träger an einem Fuß getragen wird, der in dem ersten Oberteil (1214) aufgenommen ist, der dritte Markierungsabschnitt (1246, 1248) im Wesentlichen mit einem oder mehreren Merkmalen des ersten Markierungsabschnitts (1242) auf dem ersten Oberteil (1214) ausgerichtet ist, um eine zweite Markierung zu bilden, die sich von der ersten Markierung unterscheidet.

8. System nach Anspruch 1, wobei das System überdies einen zweiten Schuhartikel (1611; 1710) umfasst, der mit dem ersten Schuhartikel (1610) austauschbar ist;
wobei der zweite Schuhartikel (1611, 1710) eine zweite Sohlenstruktur (1613; 1712) und ein zweites Oberteil (1614; 1714) umfasst, das an der zweiten Sohlenstruktur (1613; 1712) gesichert ist, wobei das zweite Oberteil (1615; 1714) eine zweite Öffnung (1623; 1722) bestimmt, die ausgestaltet ist, einen Fuß eines Trägers aufzunehmen, wobei das zweite Oberteil (1615; 1714) einen dritten Markierungsabschnitt (1644; 1740) umfasst, der benachbart zur zweiten Öffnung (1623; 1722) angeordnet ist, wobei der dritte Markierungsabschnitt (1644; 1740) ausgestaltet ist, mit dem zweiten Markierungsabschnitt (1642) auf der Socke (1638) ausgerichtet zu sein, wenn die Socke (1638) und der zweite Schuhartikel (1611; 1710) vom Träger getragen werden; und
wobei die zweite Öffnung (1623; 1722) des zweiten Schuhartikels (1611; 1710) ausgestaltet ist, eine andere Menge des zweiten Markierungsabschnittes (1642) zu enthüllen, wenn er vom Träger getragen wird, als dies beim ersten Schuhartikel (1610) der Fall ist.

9. System nach Anspruch 8, wobei der dritte Markierungsabschnitt im Wesentlichen der erste Markierungsabschnitt (136) ist; und
wobei, wenn die Socke (1638) vom Träger an einem Fuß getragen wird, der in dem zweiten Oberteil (1714) des zweiten Schuhartikels (1710) aufgenommen ist, der dritte Markierungsabschnitt (1740) mit dem zweiten Markierungsabschnitt (1642) ausgerichtet ist, um eine zweite Markierung zu bilden, die sich von der ersten Markierung unterscheidet.

## Revendications

1. Système de chaussures (105) comportant :
un premier article de chaussures (110) incluant une première structure de semelle (112) et une première tige (114) fixée à la première structure de semelle (112), le premier article de chaussures (110) ayant une région d'avant-pied (116), une région de milieu de pied (118) et une région de talon (120), la première tige (114) définissant une première ouverture (122) conçue pour recevoir un pied d'un utilisateur, dans lequel la première tige (114) inclut une première portion de repère (136) disposée adjacente à la première ouverture (122) ; et
une chaussette incluant ainsi une deuxième portion de repère (140), dans lequel une ou plusieurs caractéristiques de ladite deuxième portion de repère (140) sont adaptées pour être sensiblement alignées sur la ou les caractéristiques de la première portion de repère (136) sur la tige afin de former un premier repère (134) lorsque la chaussette (138) est portée par l'utilisateur sur un pied reçu au sein de la tige (114) ;
dans lequel la première portion de repère (136) inclut une première paire de marquages allongés (142) disposés sur un arrière d'une région de talon de la tige (114) et s'étendant dans une direction sensiblement verticale ;
dans lequel la deuxième portion de repère (140) disposée sur la chaussette (138) inclut une deuxième paire de marquages allongés (144) s'étendant vers le haut et vers un côté médian (148) de la chaussette (138) et une troisième paire de marquages allongés (146) s'étendant vers le haut et vers un côté latéral (150) de la chaussette (138) ;
dans lequel la première paire de marquages allongés (142) inclut un premier marquage (152) et un deuxième marquage (154) orienté sensiblement parallèle au premier marquage (152) ;
dans lequel la deuxième paire de marquages allongés (144) inclut un troisième marquage (156) et un quatrième marquage (158) orienté sensiblement parallèle au troisième marquage (156) ;
dans lequel la troisième paire de marquages allongés (146) inclut un cinquième marquage (160) et un sixième marquage (162) orienté sensiblement parallèle au cinquième marquage (160) ;
dans lequel chaque paire de marquages allongés (142, 144, 146) est conçue pour s'étendre sensiblement radialement à partir d'une région centrale (165) ;
dans lequel le premier marquage (152) inclut un premier bord intérieur (170) ;
dans lequel le deuxième marquage (154) inclut un second bord intérieur (172) ;
dans lequel le premier bord intérieur (170) est sensiblement parallèle au second bord intérieur (172) ;
dans lequel le premier bord intérieur (170) du premier marquage (152) est en alignement substantiel avec un troisième bord (178) du cinquième marquage (160) ;
dans lequel le second bord intérieur (172) du deuxième marquage (154) est en alignement substantiel avec un quatrième bord (180) du troisième marquage (156) ; et
dans lequel la chaussette (138) inclut une région d'avant-pied, une région de milieu de pied et une région de talon.

2. Système (105) selon la revendication 1, dans lequel, lorsque la chaussette (138) est portée par l'utilisateur sur un pied reçu au sein de la tige (114), une ou plusieurs caractéristiques de la deuxième portion de repère (140) sont sensiblement non alignées avec une ou plusieurs caractéristiques de la première portion de repère (136) sur la tige (114).

3. Système selon la revendication 1, dans lequel le système (1105) inclut une paire de chaussures incluant une chaussure gauche (1110) conçue pour recevoir un pied gauche de l'utilisateur et une chaussure droite (1111) conçue pour recevoir le pied droit de l'utilisateur, le système (1105) incluant en outre une paire de chaussettes incluant une chaussette gauche (1138) conçue pour recevoir un pied gauche de l'utilisateur et une chaussette droite (1139) conçue pour recevoir le pied droit de l'utilisateur, dans lequel la chaussette gauche (1138) inclut une première section (1144) du premier repère (1136) et la chaussette droite (1139) inclut une deuxième section (1145) du premier repère (1136) et dans lequel la première section (1144) du premier repère (1136) est différente de la deuxième section (1145) du premier repère (1136).

4. Système selon la revendication 3, dans lequel, lorsque la chaussette gauche (1138) et la chaussette droite (1139) sont portées par l'utilisateur, la première section (1144) du premier repère (1136) et la deuxième section (1145) du premier repère (1136) s'alignent sensiblement l'une sur l'autre pour former une sous-portion du premier repère (1136).

5. Système selon la revendication 1, dans lequel le système (1105) inclut une paire de chaussures incluant une chaussure gauche (1110) conçue pour recevoir le pied gauche de l'utilisateur et une chaussure droite (1111) conçue pour recevoir le pied droit de l'utilisateur, le système (1105) incluant en outre une chaussette gauche (1138) conçue pour recevoir le pied gauche de l'utilisateur et une chaussette droite (1139) conçue pour recevoir le pied droit de l'utilisateur ;
dans lequel la chaussette gauche (1138) inclut une première section (1144) du premier repère (1136) et la chaussette droite (1139) inclut une seconde section (1145) du premier repère (1136) ; et
dans lequel la chaussure gauche (1110) inclut une troisième section (1142) du premier repère (1136) et la chaussure droite (1111) inclut une quatrième section (1143) du premier repère (1136) et dans lequel la troisième section (1142) du premier repère (1136) est différente de la quatrième section (1143) du premier repère (1136).

6. Système selon la revendication 5, dans lequel la première section (1144) du premier repère (1136), la deuxième section (1145) du premier repère (1136), la troisième section (1142) du premier repère (1136) et la quatrième section (1143) du premier repère (1136) s'alignent sensiblement l'une sur l'autre pour former le premier repère (1136).

7. Système selon la revendication 1, dans lequel la chaussette est une première chaussette (1238) et le système (1205) inclut en outre une seconde chaussette (1239, 1240) qui est interchangeable avec la première chaussette (1238) ;
dans lequel la seconde chaussette (1239, 1240) inclut une troisième portion de repère (1246, 1248) ; et
dans lequel, lorsque la seconde chaussette (1239, 1240) est portée par l'utilisateur sur un pied reçu au sein de la première tige (1214), la troisième portion de repère (1246, 1248) s'aligne sensiblement sur une ou plusieurs caractéristiques de la première portion de repère (1142) sur la première tige (1214) pour former un second repère qui est différent du premier repère.

8. Système selon la revendication 1, dans lequel le système inclut en outre un second article de chaussures (1611 ; 1710) qui est interchangeable avec le premier article de chaussures (1610) ;
le second article de chaussures (1611 ; 1710) incluant une seconde structure de semelle (1613 ; 1712) et une seconde tige (1614; 1714) fixée à la seconde structure de semelle (1613 ; 1712), la seconde tige (1615 ; 1714) définissant une seconde ouverture (1623 ; 1722) conçue pour recevoir un pied d'un utilisateur, dans lequel la seconde tige (1615 ; 1714) inclut une troisième portion de repère (1644 ; 1740) disposée adjacente à la seconde ouverture (1623 ; 1722), la troisième portion de repère (1644 ; 1740) étant conçue pour s'aligner sur la seconde portion de repère (1642) sur la chaussette (1638) lorsque la chaussette (1638) et le second article de chaussures (1611 ; 1710) sont portés par l'utilisateur ; et
dans lequel la seconde ouverture (1623 ; 1722) du second article de chaussures (1611 ; 1710) est conçue pour exposer une quantité différente de la deuxième portion de repère (1642) lorsqu'il est porté par l'utilisateur par rapport au premier article de chaussures (1610).

9. Système selon la revendication 8, dans lequel la troisième portion de repère est sensiblement la même que la première portion de repère (136) ; et
dans lequel, lorsque la chaussette (1638) est portée par l'utilisateur sur un pied reçu au sein de la seconde tige (1714) du second article de chaussures (1710), la troisième portion de repère (1740) s'aligne sur la deuxième portion de repère (1642) afin de former un second repère qui est différent du premier repère.
